# EUROPEAN PATENT APPLICATION

(11) **EP 1 553 185 A1**
(43) Date of publication of application: **13.07.2005**
(21) Application number: 03771343.5
(22) Date of filing: 25.07.2003
(51) Int. Cl.: C12P 7/66

(54) **PROCESS FOR PRODUCING SOLUTION CONTAINING UBIQUINONE-10**

(30) Priority: 25.07.2002 JP 2002217159
(71) Applicant: KYOWA HAKKO KOGYO CO., LTD., Chiyoda-ku, Tokyo 100-8185 (JP)
(72) Inventor: MURATA, Hideki,c/o Technical Research Laboratories, Hofu-shi, Yamaguchi 747-8522 (JP); YONEMITSU, Hiroyuki, Cape Girardeau, MO 63701 (US)
(74) Representative: Williams, Aylsa
(86) International application number: PCT/JP2003/009459
(87) International publication number: WO 2004/011660

(57) **Abstract**

In accordance with the present invention, a process for producing a ubiquinone-10-containing solution is provided, which comprises the following steps:
[1] adding a methanol solution to a culture obtained by culturing a microorganism having an ability to produce ubiquinone-10 in a medium, a processed product of the culture, or a partially purified product of ubiquinone-10, to a final concentration of 50 to 100 v/v% and then retaining the resulting mixture at a temperature of 0°C or above to 30°C or below;
[2] separating and recovering an insoluble matter from the solution obtained at the step [1];
[3] adding a methanol solution of a concentration of 85 to 100 v/v% to the insoluble matter obtained in the step [2] and retaining the resulting mixture at a temperature of more than 30°C and 80°C or below; and
[4] removing an insoluble matter from the solution obtained in the step [3].

## Description

### Technical Field

The present invention relates to a process for separating and purifying ubiquinone-10 from a culture containing ubiquinone-10, a processed product of the culture, or a partially purified product of ubiquinone-10.

### Background Art

Ubiquinone-10 is widely distributed in the tissues of animals and plants, and microbial cells, and plays an important role as an essential component of the peripheral electron transport system. Further, the pharmacological action thereof is effective for congestive heart failure and coronary failure, muscular dystrophy due to nutritional disorders, and the like. Ubiquinone-10 is a highly valuable substance as apharmaceutical product.

As the method for producing ubiquinone-10, a method which comprises culturing a microorganism having a high ubiquinone-10 content and extracting ubiquinone-10 from a resulting culture is prevarent.

As the method for purifying ubiquinone-10 from the culture, an extraction method using organic solvents is known (Japanese Published Unexamined Patent Application no.178595/99, etc.). As the method for purifying ubiquinone-10 from the extract solution, a method using silica gel or active alumina (Japanese Published Unexamined Patent Application no.91360/88, Japanese Published Unexamined Patent Application no.160953/89, etc.) is known.

However, the extract solution obtained by the extraction method described in the above contains a high content of ubiquinone-10 analogs other than ubiquinone-10. Therefore, it is very difficult to purify ubiquinone-10 having high purity, directly from the extract solution by a crystallization.

According to the method using silica gel or active alumina, ubiquinone-10 cannot efficiently be separated or purified in a case where an extract solution containing a high content of ubiquinone-10 analogs is used. Further, silica gel and active alumina are expensive and disadvantageously involve cost highly for the production at an industrial scale.

### Disclosure of the Invention

It is an object of the present invention to provide a method for separating and purifying ubiquinone-10 from a culture containing ubiquinone-10, a processed product of the culture, or a partially purified product of ubiquinone-10 at low cost.

The present invention relates to those described in (1) to (6) below.
(1) A process for producing a ubiquinone-10-containing solution, which comprises the following steps:
   [1] adding a methanol solution to a culture obtained by culturing a microorganism having an ability to produce ubiquinone-10 in a medium, a processed product of the culture, or a partially purified product of ubiquinone-10, to a final concentration of 50 to 100 v/v% and then retaining the resulting mixture at a temperature of 0°C or above and 30°C or below;
   [2] separating and recovering an insoluble matter from the solution obtained at the step [1];
   [3] adding a methanol solution of a concentration of 85 to 100 v/v% to the insoluble matter obtained in the step [2] and retaining the resulting mixture at a temperature of more than 30°C and 80°C or below; and
   [4] a step of removing an insoluble matter from the solution obtained in the step [3].
(2) The process according to the above (1), where in the steps of adding a methanol solution again to the insoluble matter obtained in the step [2] to a final concentration of 50 to 100 v/v%, retaining a resulting mixture at a temperature of 0°C or above and 30°C or below, and subsequently separating and recovering a resulting insoluble matter are repeated once or more times before the subsequent step [3].
(3) The process according to the above (1) or (2), wherein the microorganism having an ability to produce ubiquinone-10 is a microorganism selected from microorganisms of basidiomycetes, fungi, yeast and bacteria.
(4) The process according to the above (1) or (2), wherein the processed product of the culture is a concentrate of the culture of the microorganism, a dried product of the culture, a bacterial cell obtained by separation from the culture, a dried product of the bacterial cell, a freeze-dried product of the bacterial cell, a rinsed bacterial cell obtained by rinsing the bacterial cell, a dried product of the rinsed bacterial cell or a freeze-dried product of the rinsed bacterial cell.
(5) A process for producing the crystal of ubiquinone-10, which comprises depositing the crystal of ubiquinone-10 from the ubiquinone-10-containing solution obtained by the process according to the above (1) or (2).
(6) The process according to the above (5), wherein the crystal of ubiquinone-10 is a crystal having a purity of 90.0% or more.

As the microorganism having an ability to produce ubiquinone-10 for use in the process of the present invention, any microorganism can be used as long as it has such ability. By way of example, microorganisms of Basidiomycetes, fungi, yeast and bacteria are known as microorganisms capable of producing ubiquinone-10. More specifically, the microorganisms of Basidiomycetes include those of genus Ustilago; the fungi include those of genera Aspergillus, Exobasidium, Geotrichum, Monascus, Paecilomyces, Sporotrichum and Tilletiopsis; yeast includes those of genera Aureobasidium, Brettanomyces, Bullera, Candida, Cryptococcus, Leucosporidium, Oosporidium, Rhodotorula, Rhodosporium, Schizosaccharomyces, Sporobolomyces, Torulopsis, Tremella, Tichosporon and Sporidiobolus; and bacteria include those of genera Acetobacter, Agrobacterium, Corynebacterium, Erythrobacter, Flavobacterium, Methylobacter, Microcyclus, Paracoccus, Phyllobacterium, Protaminobacter, Pseudomonas, Rhizobium, Rhodobacter and Xanthomonas.

Additionally, microorganisms belonging to the genus Escherichia with ubiquinone synthesizing enzyme enhanced by the method such as gene recombination, the microorganisms described in the above which have the ability to produce ubiquinone-10 with the enhanced enzyme may also be used for the process of the present invention.

As the medium for culturing the microorganisms, any culture media such as natural medium and synthetic medium may satisfactorily be used, as long as the culture media contain carbon sources, nitrogen sources and inorganic salts assimilable by the microorganisms, to efficiently culture the microorganisms therein.

As the carbon sources, any carbon source assimilable by the microorganisms may be satisfactory. As such, glucose, fructose, sucrose and molasses containing them, carbohydrates such as starch or starch hydrolyzates, organic acids such as acetic acid and propionic acid, and alcohols such as ethanol and propanol can be used.

As the nitrogen sources, ammonia, ammonium salts with inorganic acids or organic acids, such as ammonium chloride, ammonium sulfate, ammonium acetate, and ammonium phosphate, other nitrogen-containing compounds, peptone, meat extract, yeast extract, corn steep liquor, casein hydrolyzates, soybean bran, soybean bran hydrolyzates, various fermenting bacterial cells and digestion products thereof can be used.

As the inorganic salts, potassium hydrogen phosphate, dipotassium hydrogen phosphate, magnesium phosphate, magnesium sulfate, sodium chloride, ferrous sulfate, manganese sulfate, copper sulfate, and calcium carbonate can be used.

Culturing is carried out under aerobic conditions, for example by agitation culture or submerged aeration agitation culture. The temperature for culturing is preferably 15 to 40°C, while the time period for culturing is generally 16 hours to 14 days. During culturing, the pH is preferably retained at pH 3.0 to 9.0. The pH is adjusted, with for example inorganic or organic acids, alkaline solutions, urea, calcium carbonate and ammonia.

On completion of culturing, the resulting culture can be used as it is for the purification according to the present invention. A processed product of the culture can be used for the purification according to the present invention as well.

The processed product of the culture includes for example a concentrate of the culture, a dried product of the culture, a bacterial cell obtained by separation from the culture, a dried product of the bacterial cell, a freeze-dried product of the bacterial cell, a rinsed bacterial cell obtained by rinsing the bacterial cell, a dried product of the rinsed bacterial cell or a freeze-dried product of the rinsed bacterial cell.

The rinsed bacterial cell means a rinsed bacterial cell obtained by rinsing the bacterial cell with a solvent substantially incapable of dissolving ubiquinone-10 and includes for example a bacterial cell obtained by rinsing the bacterial cell once to 10 times, preferably two to seven times, more preferably three to five times, using the solvent.

As the solvent for use in rinsing described above, water is preferably used.

In accordance with the present invention, further, the phrase "substantially incapable of dissolving ubiquinone-10" means dissolving ubiquinone-10 at a level acceptable for the industrial purification process of ubiquinone-10. Specifically, it means that the solvent has a ubiquinone-10 solubility at 0.05% or less, preferably 0.02% or less, more preferably 0.01% or less.

The bacterial cells of microorganisms can be obtained by separation procedures such as filtration, centrifugation and membrane separation, preferably by centrifugation. Filtration can be carried out with Nutsche, filter press, basket separator and the like.

The culture and the proces sed product thereof can be frozen and stored, and thawed for use if necessary.

According to the present invention, ubiquinone-10 can be purified from the culture and the processed product thereof and additionally from a partially purified product of ubiquinone-10.

The partially purified product of ubiquinone-10 includes for example ubiquinone-10-containing solutions, dried products, freeze-dried products or crystallized products having high contents of ubiquinone-10 analogs. Any method is satisfactory as the method for obtaining these products. The method includes for example a method of extracting ubiquinone-10 from a culture obtained by culturing a microorganism having an ability to produce ubiquinone-10 according to conventional methods, using an organic solvent or the like, a method of drying or freeze-drying the extract solution, and a method of crystallizing the extract solution obtained by the crude extraction method.

The ubiquinone-10-containing solutions having high contents of ubiquinone-10 analogs include for example a ubiquinone-10-containing solution containing ubiquinone-10 analogs in 5 parts by weight or more to 95 parts by weight of ubiquinone-10.

The ubiquinone-10 analogs include for example structurally analogous ubiquinone-10 analogs such as 3-demethoxy ubiquinone-10 and carotenoids such as spheroidene.

The methanol solution for use in the process of the present invention includes methanol and aqueous methanol solution. Methanol solutions prepared by adding other organic solvents and the like to methanol are also included in the methanol solution for use in the process of the present invention, as long as the methanol solutions can achieve the object of the present invention.

By adding the methanol solution to the culture of the microorganism or a processed product thereof, or a pattially purified product of ubiquinone-10 to a final concentration of 50 to 100 v/v% and then retaining the resulting mixture at a temperature of 0°C or above and 30°C or below, an insoluble matter containing ubiquinone-10 having a high content can be obtained. The concentration of the methanol solution and the temperature thereof may satisfactorily be a combination of any concentration and any temperature, as long as the methanol solution at the combination of the concentration and the temperature substantially incapable of dissolving ubiquinone-10. In a case where methanol or aqueous methanol solution is for example used as the methanol solution, a combination of a concentration of 50 to 100 v/v% and a temperature of 0 to 30°C, a preferable combination of a concentration of 70 to 100 v/v% and a temperature of 10 to 20°C and a more preferable combination of a concentration of 80 to 100 v/v% and a temperature of 20°C can be listed.

As the method for retaining a solution containing the culture or the like at the concentration and the temperature, a method of agitation with an agitator for 30 minutes to 10 hours, preferably one to 5 hours, more preferably one to 2 hours is exemplified. By the method, ubiquinone-10 analogs contained in the culture or the like except ubiquinone-10 can be extracted in the methanol solution, to obtain an insoluble matter containing ubiquinone-10.

The insoluble matter containing ubiquinone-10 as obtained in the step described above can be separated from the solution by separation procedures such as filtration, centrifugation and membrane separation. Filtration can be carried out with Nutsche, filter press, basket separator and the like.

By adding again the methanol solution to the insoluble matter obtained by the series of steps described above to a concentration of 50 to 100 v/v% and repeating the series of steps described above once or more, for example several times, an insoluble matter containing an intended concentration of ubiquinone-10 may satisfactorily be obtained.

By adding a methanol solution of a concentration of 85 to 100 v/v% to the insoluble matter containing ubiquinone-10 as obtained in the aforementioned step, and then retaining the mixture at a temperature of more than 30°C and 80°C or below, a methanol solution containing ubiquinone-10 can be recovered.

The concentration of the methanol solution to be added to the insoluble matter and the temperature thereof may be any combination of any concentration and any temperature, as long as the methanol solution at the concentration and at the temperature dissolves ubiquinone-10 contained in the insoluble matter separated and recovered at the aforementioned step but substantially incapable of dissolving residual matters except ubiquinone-10. Specifically, for example, a combination of the concentration of the methanol solution at 85 to 100 v/v% and the temperature thereof at more than 30°C and 80°C or below, a preferable combination of the concentration of the methanol solution at 90 to 100 v/v% and the temperature at 50 to 70°C, and a more preferable combination of the concentration of the methanol solution at 95 to 100 v/v% and the temperature at 60 to 70°C are listed.

The method for retaining the methanol solution at the concentration and the temperature includes for example a method with agitation with an agitator for 30 minutes to 10 hours, preferably for one to 5 hours and more preferably for one to 2 hours. According to the method, a methanol solution containing ubiquinone-10 can be obtained.

By removing insoluble matters from the methanol solution containing ubiquinone-10 as obtained in the aforementioned step, a ubiquinone-10-containing solution can be separated and obtained. The method for removing insoluble matters includes a method of separating the insoluble matters from the solution by separation procedures such as filtration, centrifugation, and membrane separation. Filtration can be carried out with Nutsche, filter press, basket separator and the like.

By depositing the crystal of ubiquinone-10 from the ubiquinone-10-containing solution obtained in the step described above by crystallization and the like, the crystal of ubiquinone-10 can be recovered.

The process of crystallization for use according to the present invention includes any process by which the crystal of ubiquinone-10 can be deposited from the ubiquinone-10-containing solution obtained by the method of the present invention. The process includes for example concentration crystallization process, cooling crystallization process and combination processes thereof. Any crystallization conditions may be satisfactory as the crystallization conditions, as long as the crystal of ubiquinone-10 can be crystallized under the conditions. The conditions can be preset by a person skilled in the art, with no error or mistake. The crystallization conditions for the cooling crystallization process are as follows. The ubiquinone-10-containing solution obtained in the step is cooled to 0 to 30°C, preferably 10 to 25°C, more preferably 15 to 20°C over one to 30 hours, preferably 2 to 20 hours, more preferably 5 to 15 hours.

The crystal deposited by the crystallization process is separated from the solution by filtration, centrifugation or membrane separation and is then dried, to obtain the crystal of ubiquinone-10. Adding a methanol solution to the resulting crystal to a concentration of 85 to 100 v/v% and then retaining the solution at a temperature of more than 30°C and less than 80°C to dissolve the crystal, the crystallization process is repeatedly carried out once or more, for example several times, to increase the purity of the resulting crystal.

The crystal of ubiquinone-10 obtained according to the present invention includes a crystal of ubiquinone-10 at a ubiquinone-10 purity having 90.0% or more, preferably 95.0% ormore, more preferably 97 . 0% ormore, and still more preferably 99.0% or more.

### Brief Description of the Drawings

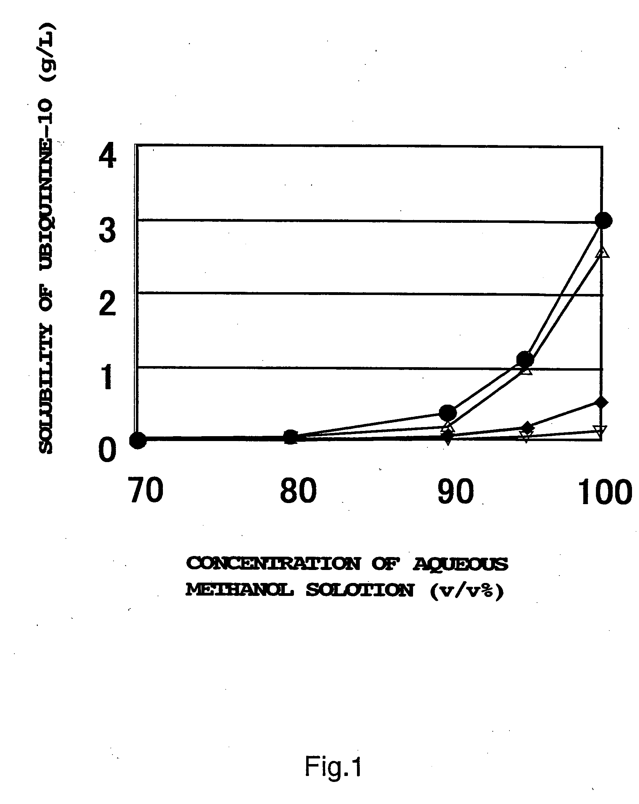

Fig.1 depicts the ubiquinone-10 solubility in aqueous methanol solutions. In the figure, the solubility of ubiquinone-10 is shown at temperatures of aqueous methanol solutions, which was marked with solid circles at 70°C; with open triangles at 50°C; with solid diamonds at 30°C; and with inversed open triangles at 20°C.

The Examples of the present invention are now shown below. These examples are not to be construed as limiting the scope of the present invention.

### Best Mode for Carrying out the Invention

### Example 1

### Process of efficiently removing ubiquinone-10 analogs from a mixture of ubiquinone-10 and ubiquinone-10 analogs

A medium consisting of the composition shown below in Table 1 was adjusted to pH 9.0, to which calcium carbonate was added at 1%. Subsequently, the medium was sterilized at 121°C for 10 minutes. 1.8 liters of the resulting medium were placed in a 3-liter fermentation tank, where Rhodobacter sphaeroides ATCC 21286 as a bacterium having an ability to produce ubiquinone-10 was inoculated for culturing at an agitated rotation number of 450 rpm and at 28°C for 8 days. In the table, the trace element means a solution composed of 88 mg/l sodium tetraborate (borax: Na₂B₄O₇ · 10H₂O), 37 mg/l ammonium molybdate [(NH₄)₆Mo₇O₂₄ · 4H₂O], 8.8 mg/l zinc sulfate (ZnSO₄), 270 mg/l copper sulfate (CuSO₄ · 5H₂O), 7.2 mg/l manganese chloride (MnCl₂ · 4H₂O) and 970 mg/l ferric chloride (FeCl₃ · 6H₂O).

**Table 1**

| Composition | Concentration |
|---|---|
| Molasses | 4.0% |
| Glucose | 2.7% |
| Corn steep liquor | 4.0% |
| Ammonium sulfate | 0.8% |
| Potassium dihydrogenphosphate | 0.05% |
| Dipotassium hydrogenphosphate | 0.05% |
| Magnesium sulfate · 7 H₂O | 0.025% |
| Ferrous sulfate · 7H₂O | 3.0 mg/l |
| Thiamine | 8.0 mg/l |
| Nicotinic acid | 8.0 mg/l |
| Trace element | 1.0 ml/l |

After completion of culturing, 1 µl of 20% potassium ferricyanide [K₃Fe(CN)₆] solution, 0.3 ml of 2-butanol and 0.3 ml of glass beads were added to 0.3 ml of the culture, stirred with a multibeads shocker MB200 (manufactured by Yasui Machine Co., Ltd.) for 30 minutes to disrupt the bacterial cell, to thoroughly extract ubiquinone-10 and ubiquinone-10 analogs. The extract solution obtained by disruption was centrifuged at 15,000 rpm for 10 minutes, to recover the resulting supernatant, which was then analyzed by HPLC. Consequently, the ratio of 3-demethoxy ubiquinone-10 to ubiquinone-10 was 1%.

After completion of culturing, alternatively, the culture was centrifuged to collect a 80 g of wet bacterial cell, which was then rinsed three times with water, to obtain a rinsed bacterial cell. 500 ml of 100 v/v% methanol at 10, 20, 40 or 60°C was added to the rinsed bacterial cell, for agitation for one hour. Then, the resulting mixture was centrifuged to recover the precipitate. The ratio of 3-demethoxy ubiquinone-10 (3-dmUBD) to ubiquinone-10 in the resulting precipitate was analyzed by high-performance liquid chromatography. The results are shown in Table 2.

**Table 2**

| Temperature (°C) | 3-dmUBD ratio (%) |
|---|---|
| 10 | 0.03 |
| 20 | 0.05 |
| 40 | 0.71 |
| 60 | 0.61 |

Base on the results, it was confirmed that ubiquinone-10 analogs could be efficiently removed from the solution containing ubiquinone-10 and ubiquinone-10 analogs, according to the process of the present invention.

### Example 2

### Solubility of ubiquinone-10 in methanol solution

Using a standard ubiquinone-10 (manufactured by Wako Pure Chemical Industries Co., Ltd.), the solubility of ubiquinone-10 in an aqueous methanol solution was examined in terms of the relation between the concentration of the solution and temperature. The results are shown in Fig.1.

It was shown that the solubility of ubiquinone-10 increased in proportion to the methanol concentration and the temperature of the aqueous methanol solution.

### Example 3

### Purification of ubiquinone-10 from a bacterial cell of a ubiquinone-10-producing bacterium

80 g of a wet bacterial cell of Rhodobacter sphaeroides ATCC21286 as obtained by the same method as in Example 1 was rinsed three times with water, to obtain a rinsed bacterial cell. 500 ml of methanol was added as an extraction solvent to the rinsed bacterial cell, for agitation at 20°C for one hour and subsequent centrifugation, to discard a methanol solution phase containing contaminants at a high content. The methanol extraction procedure was repeated twice for the resulting precipitate.

Subsequently, methanol was added again for agitation at 60°C for one hour, an extract solution was obtained through filtration. The extract solution contained 99 parts by weight of ubiquinone-10 to one part by weight of 3-demethoxyubiquinone-10.

By cooling the extract solution to 20°C over 5 hours or more, ubiquinone-10 was deposited, to obtain a crude crystal of ubiquinone-10. The crystal was dissolved in methanol at 40°C to 0.5 g/liter, and the resulting solution was cooled to 20°C over 5 hours, to obtain a crystal of ubiquinone-10. The purity of the crystal was 99.5%.

### Example 4

### Purification of ubiquinone-10 from a dried bacterial cell of a ubiquinone-10-producing bacterium

Water was added to 80 g of a wet bacterial cell obtained by centrifuging a culture obtained by the same method as in Example 1, to allow the bacterial cell to resume a slurry state, and thus a dried bacterial cell was obtained with a spray dryer (at a water content of 2.0 w/w%). To the dried bacterial cell was added 500 ml of methanol as an extraction solvent, for agitation at 20°C for one hour, the resulting mixture was centrifuged to remove the methanol solution phase. The methanol extraction procedure was repeated twice for the resulting precipitate.

Subsequently, methanol was added again for agitation at 60°C for one hour, an extract solution was obtained through filtration. The extract solution contained 99 parts by weight of ubiquinone-10 to one part by weight of 3-demethoxyubiquinone-10.

By cooling the extract solution to 20°C over 5 hours or more, ubiquinone-10 was deposited, to obtain a crude crystal of ubiquinone-10. The crystal was dissolved in methanol at 40°C to 0.5 g/liter, and the resulting solution was cooled to 20°C over 5 hours or more, to obtain a crystal of ubiquinone-10. The purity of the crystal was 99.5%.

### Example 5

### Purification of ubiquinone-10 from a culture of a ubiquinone-10-producing bacterium

0.5 liter of methanol was added to 0.5 liter of a culture obtained by the same method as in Example 1, for agitation at 20°C for one hour, and the resulting mixture was centrifuged to remove the methanol solution phase. The methanol extraction procedure described above was repeated three times for the resulting precipitate.

Subsequently, methanol was added for agitation at 60°C for one hour, an extract solutionwas obtained through filtration. The extract solution contained 99 parts by weight of ubiquinone-10 to one part by weight of 3-demethoxyubiquinone-10.

By cooling the extract solution to 20°C over 5 hours or more, ubiquinone-10 was deposited, to obtain a crude crystal of ubiquinone-10. The crystal was dissolved in methanol at 40°C to 0.5 g/liter, and the resulting solution was cooled to 20°C over 5 hours or more, to obtain a crystal of ubiquinone-10. The purity of the crystal was 99.5%.

### Example 6

### Purification of ubiquinone-10 using aqueous methanol solution

By rinsing 80 g of a wet bacterial cell obtained by centrifuging a culture obtained by the same method as in Example 1 with water, a rinsed bacterial cell was obtained. An aqueous 80 v/v% methanol solution was added to the bacterial cell for agitation at 20°C for one hour, and the resulting mixture was centrifuged to remove the methanol solution phase. The methanol extraction procedure described above was repeated five times for the resulting precipitate.

Subsequently, 95 v/v% methanol solution was added again for agitation at 60 ° C for one hour, and an extract solution was obtained through filtration. The extract solution contained 99 parts by weight of ubiquinone-10 to one part by weight of 3-demethoxyubiquinone-10.

By cooling the extract solution to 20°C over 5 hours or more, ubiquinone-10 was deposited, to obtain a crude crystal of ubiquinone-10. The crystal was dissolved in methanol at 40°C to 0.5 g/liter, and the resulting solution was cooled to 20°C over 5 hours or more, to obtain a crystal of ubiquinone-10. The purity of the crystal was 99.5%.

### Example 7

### Purification of ubiquinone-10 from a partially purified ubiquinone-10

A culture obtained by the same method as in Example 1 was centrifuged, to obtain a wet bacterial cell, from which ubiquinone-10 was extracted, using 2-butanol according to a conventional method. Ubiquinone-10 contained in the extract was adsorbed and desorbed onto or from a synthetic adsorption resin to recover a ubiquinone-10-containing fraction, which was then crystallized by concentration, to obtain a partially purified ubiquinone-10 having a purity of 82.9%. A 80 v/v% hydrous methanol solution was added to the partially purified ubiquinone-10, for agitation at 20°C for one hour, and the resulting mixture was centrifuged to remove the methanol solution phase. The methanol extraction procedure described above was repeated five times for the resulting precipitate.

Subsequently, 95 v/v% methanol solution was added again for agitation at 60 ° C for one hour, and an extract solution was obtained through filtration. The extract solution contained 99 parts by weight of ubiquinone-10 to one part by weight of 3-demethoxy ubiquinone-10.

By cooling the extract solution to 20°C over 5 hours or more, ubiquinone-10 was deposited, to obtain a crude crystal of ubiquinone-10. The crystal was dissolved in methanol at 40°C to 0.5 g/liter, and the resulting solution was cooled to 20°C over 5 hours or more, to obtain a crystal of ubiquinone-10. The purity of the crystal was 99.5%.

### Industrial Applicability

According to the process of the present invention, ubiquinone-10 having high purity can be purified at low cost from a culture of a microorganism with an ability to produce ubiquinone-10, aprocessedproduct of the culture, or a partially purified product of ubiquinone-10.

## Claims

1. A process for producing a ubiquinone-10-containing solution, which comprises the following steps:
[1] adding a methanol solution to a culture obtained by culturing a microorganism having an ability to produce ubiquinone-10 in a medium, a processed product of the culture, or a partially purified product of ubiquinone-10, to a final concentration of 50 to 100 v/v% and then retaining the resulting mixture at a temperature of 0°C or above and 30°C or below;
[2] separating and recovering an insoluble matter from the solution obtained at the step [1];
[3] adding a methanol solution of a concentration of 85 to 100 v/v% to the insoluble matter obtained in the step [2] and retaining the resulting mixture at a temperature of more than 30°C and 80°C or below; and
[4] removing an insoluble matter from the solution obtained in the step [3].

2. The process according to claim 1, wherein the steps of adding a methanol solution again to the insoluble matter obtained in the step [2] to a final concentration of 50 to 100 v/v%, retaining a resulting mixture at a temperature of 0°C or above and 30°C or below, and subsequently separating and recovering a resulting insoluble matter are repeated once or more times before the subsequent step [3].

3. The process according to claim 1 or 2, wherein the microorganism having an ability to produce ubiquinone-10 is a microorganism selected frommicroorganisms of basidiomycetes, fungi, yeast and bacteria.

4. The process according to claim 1 or 2, wherein the processed product of the culture is a concentrate of the culture of the microorganism, a dried product of the culture, a bacterial cell obtained by separation from the culture, a dried product of the bacterial cell, a freeze-dried product of the bacterial cell, a rinsed bacterial cell obtained by rinsing the bacterial cell, a dried product of the rinsed bacterial cell or a freeze-dried product of the rinsed bacterial cell.

5. A process for producing a crystal of ubiquinone-10, which comprises depositing the crystal of ubiquinone-10 from the ubiquinone-10-containing solution obtained by the process according to claim 1 or 2.

6. The process according to claim 5, wherein the crystal of ubiquinone-10 is a crystal having a purity of 90.0% or more.
